# EUROPEAN PATENT APPLICATION

(11) **EP 4 170 677 A1**
(43) Date of publication of application: **26.04.2023**
(21) Application number: 20945918.9
(22) Date of filing: 23.07.2020
(51) Int. Cl.: G16H 50/20, G16H 30/20, G16H 30/40, G16H 50/50, G16H 50/70, G06N 3/08, G06N 20/00

(54) **DISEASE DIAGNOSIS METHOD USING NEURAL NETWORK TRAINED BY USING MULTI-PHASE BIOMETRIC IMAGE, AND DISEASE DIAGNOSIS SYSTEM PERFORMING SAME**

(71) Applicant: Deep Bio Inc., Seoul 08394 (KR)
(72) Inventor: CHO, Joon Young, Seoul 08394 (KR); KIM, Sun Woo, Seongnam-si, Gyeonggi-do 13599 (KR)
(74) Representative: Flügel Preissner Schober Seidel
(86) International application number: PCT/KR2020/009720
(87) International publication number: WO 2022/019355

(57) **Abstract**

Disclosed are a disease diagnosis method using a neural network trained by using a multi-phase biometric image, and a disease diagnosis system performing same. According to an aspect of the present invention, provided is a method comprising the steps of generating, by a diagnosis system using a neural network, a diagnosis neural network for predicting a diagnosis result regarding a predetermined disease by using a biometric image; obtaining, by the diagnosis system using the neural network, a plurality of training biometric images, wherein each of the plurality of training biometric images is labeled with a corresponding diagnosis result regarding the disease; and training, by the diagnosis system using the neural network, the diagnosis neural network by using the plurality of training biometric images, wherein the training of the diagnosis neural network comprises the steps of, for each of the plurality of training biometric images: (a) generating K noise-inserted images (here, K is an integer equal to or greater than 2) corresponding to the training biometric image by inserting noise having different characteristics into the training biometric image; and (b) training the diagnosis neural network by inputting, into the diagnosis neural network, the K noise-inserted images corresponding to the training biometric image.

## Description

### Technical Field

The present disclosure relates to a disease diagnosis system using a neural network and a method thereof. More specifically, it relates to a disease diagnosis method using a neural network trained using a multi-phase biometric image and a disease diagnosis system performing the same.

### Background Art

One of the main tasks performed by the pathology or pathology department is to read a patient's biometric slide (for example, a tissue image slide through a biopsy) and perform a diagnosis to determine the condition or symptom of a specific disease. Such diagnosis is a method that relies on the experience and knowledge of skilled medical personnel for a long time.

Recently, due to the development of machine learning, attempts to automate tasks such as recognizing or classifying images by computer systems have been actively made. In particular, attempts have been made to automate diagnosis performed by skilled medical personnel using a neural network (e.g., a deep learning method using a convolution neural network (CNN)), which is a type of machine learning.

In particular, diagnosis through deep learning using a neural network (e.g., CNN, deep learning network) does not simply automate the experience and knowledge of conventionally skilled medical personnel, and in terms of finding the characteristic elements through self-learning and deriving the desired answer, there are cases in which the characteristics of disease factors that skilled medical personnel did not know are found in the image.

In general, disease diagnosis through a neural network using a biometric image which is an image obtained by scanning a biometric slide uses a biometric image or a patch (also referred to as a tile) which is a piece of the corresponding biometric image. In other words, a medical person skilled in the biometric image or patch annotates a state of a specific disease (e.g., whether cancer has been manifested), and trains the neural network by using the plurality of annotated tiles as training data. In this case, a convolution neural network may be used as the neural network.

On the other hand, most current diagnosis methods using neural networks use already scanned data for learning and predict diagnosis results using the scanned data. In other words, the existing diagnosis method through neural network works relatively well for fixed data. However, when there is noise, it appears vulnerable. For example, in the case of image data captured in real time through a microscope, a lot of noises generated by the camera of the microscope is included in the image, and the noises of the camera itself cause a problem that the diagnosis result may continuously change.

Meanwhile, since the diagnosis speed by the neural network is generally slower than the frame rate of the camera, a problem in that the screen by the camera is cut off occurs when the part to be diagnosed through the microscope continues to move.

### [Prior Art Document]

- Patent Document : Korean Patent Application Publication No. 10-2016-0034814 "Client device with neural network and system including the same"

### Disclosure of the Invention

### Technical Goals

A technical problem to be achieved by the present disclosure is to provide a diagnosis system and method using a neural network capable of stably performing diagnosis regardless of camera noise.

In addition, it is to provide a diagnosis system and method that enables smooth screen movement by detecting movement of a camera and performing diagnosis only when a specific part is fixedly viewed without performing diagnosis when the camera is moving or when the screen is blank.

Furthermore, it is to provide a neural network structure capable of distinguishing whether a disease has onset through a specific biometric image but also a region where a disease has onset in the corresponding biometric image. In particular, it is to provide a neural network structure capable of performing segmentation for efficiently classifying regions where a disease has onset, by adding a sub-architecture for segmentation to a classification neural network architecture that may determine whether or not a disease exists in a patch.

### Technical Solutions

In accordance with an aspect of the present disclosure, there is provided a method including generating, by a diagnosis system using a neural network, a diagnostic neural network for predicting a diagnosis result related to a predetermined disease using a biometric image, acquiring, by the diagnosis system using the neural network, a plurality of biometric images for training, each of the plurality of biometric images for training being labeled with a corresponding diagnosis result for the disease, and training, by the diagnosis system using the neural network, the diagnostic neural network using the plurality of biometric images for training, wherein the training of the diagnostic neural network includes, for each of the plurality of biometric images for training, (a) generating K (where K is an integer of 2 or more) noise-inserted images corresponding to the biometric image for training by inserting noises having different characteristics into the biometric image for training, and (b) training the diagnostic neural network by inputting the K noise-inserted images corresponding to the biometric image for training to the diagnostic neural network.

In an embodiment, the operation (b) may include generating one training data corresponding to the biometric image for training by concatenating all the K noise-inserted images corresponding to the biometric image for training, the training data corresponding to the biometric image for training being labeled with the diagnosis result for the biometric image for training, and training the diagnostic neural network by inputting the training data corresponding to the biometric image for training to the diagnostic neural network.

In an embodiment, the method may further include a diagnosis operation, and the diagnosis operation may include acquiring K diagnostic object biometric images continuously photographed by an image sensor, and predicting a diagnosis result for the disease by inputting the K diagnostic object biometric images into the diagnostic neural network.

In an embodiment, the diagnosis operation may further include determining whether the image sensor is moving based on the K diagnostic object biometric images, and the predicting of the diagnosis result for the disease by inputting the K diagnostic object biometric images into the diagnostic neural network may include, when it is determined that the image sensor is not moving, predicting the diagnosis result for the disease by inputting the K diagnostic object biometric images into the diagnostic neural network.

In an embodiment, the method may further include a diagnosis operation, and the diagnosis operation may include acquiring K diagnostic object biometric images continuously photographed by an image sensor, generating one diagnostic object data by concatenating all the K diagnostic object biometric images, and predicting a diagnosis result for the disease by inputting the diagnostic object data into the diagnostic neural network.

In an embodiment, the diagnostic neural network may be a segmentation neural network configured to receive the diagnostic object data in an input layer and specify a region in which the disease exists in the diagnostic object biometric image, and the segmentation neural network may include a classification neural network configured to receive the diagnostic object data in an input layer and output a classification result on whether the disease exists in the biometric image, and a segmentation architecture configured to receive a feature map generated from each of two or more feature map extraction layers among hidden layers included in the classification neural network and specify a region in which the disease exists in the biometric image.

In an embodiment, the segmentation architecture may include a convolution sub-architecture comprising convolution nodes corresponding to the two or more feature map extraction layers, respectively, each of the convolution nodes performing convolution on a feature map input from the corresponding feature map extraction layer or two or more different convolutions, and a segmentation sub-architecture configured to specify a region in which the disease exists in a patch based on a convolution result generated by the convolution sub-architecture.

In an embodiment, the disease may be prostate cancer.

In accordance with another aspect of the present disclosure, there is provided a computer program recorded on a non-transitory computer-readable medium for performing the method described above, which is installed in a data processing device.

In accordance with another aspect of the present disclosure, there is provided a diagnosis system using a neural network, including a processor, and a memory configured to store a computer program, wherein the computer program, when executed by the processor, causes the diagnosis system using the neural network to perform the method described above.

In accordance with another aspect of the present disclosure, there is provided a diagnosis system using a neural network, including a storage module configured to store a diagnostic neural network for predicting a diagnosis result related to a predetermined disease using a biometric image, an acquisition module configured to acquire a plurality of biometric images for training, each of the plurality of biometric images for training being labeled with a corresponding diagnosis result for the disease, and a learning module configured to train the diagnostic neural network using the plurality of biometric images for training, wherein the learning module performs, for each of the plurality of biometric images for training, (a) generating K (where K is an integer of 2 or more) noise-inserted images corresponding to the biometric image for training by inserting noises having different characteristics into the biometric image for training, and (b) training the diagnostic neural network by inputting the K noise-inserted images corresponding to the biometric image for training to the diagnostic neural network.

In an embodiment, the operation (b) may include generating one training data corresponding to the biometric image for training by concatenating all the K noise-inserted images corresponding to the biometric image for training, the training data corresponding to the biometric image for training being labeled with the diagnosis result for the biometric image for training, and training the diagnostic neural network by inputting the training data corresponding to the biometric image for training to the diagnostic neural network.

In an embodiment, the diagnosis system may further include a diagnosis module configured to acquire K diagnostic object biometric images continuously photographed by an image sensor and predict a diagnosis result for the disease by inputting the K diagnostic object biometric images into the diagnostic neural network.

In an embodiment, the diagnosis module may be configured to determine whether the image sensor is moving based on the K diagnostic object biometric images, and when it is determined that the image sensor is not moving, predict the diagnosis result for the disease by inputting the K diagnostic object biometric images into the diagnostic neural network.

In an embodiment, the diagnosis system using a neural network may further include a diagnosis module configured to acquire K diagnostic object biometric images continuously photographed by an image sensor, generate one diagnostic object data by concatenating all the K diagnostic object biometric images, and predict a diagnosis result for the disease by inputting the diagnostic object data into the diagnostic neural network.

### Advantageous Effects

According to the technical idea of the present disclosure, it is possible to provide a diagnosis system and method using a neural network capable of stably performing diagnosis regardless of camera noises.

In addition, it is possible to provide a diagnosis system and method that enables smooth screen movement by detecting movement of a camera and performing diagnosis only when a specific part is fixedly viewed without performing diagnosis when the camera is moving or when the screen is blank.

In addition, it is possible to provide an efficient neural network structure that may perform not only classification for determining whether a disease has onset for each biometric image, but also segmentation for distinguishing regions where a disease has onset in the corresponding biometric image.

### Brief Description of Drawings

In order to more fully understand the drawings cited in the detailed description of the present disclosure, a brief description of each drawing is provided.
FIG. 1 is a diagram illustrating an operating environment of a method of training a neural network using a multi-phase biometric image and diagnosing a disease using the same according to the technical idea of the present disclosure.
FIG. 2 is a diagram illustrating a schematic configuration of a disease diagnosis system in accordance with an embodiment of the present disclosure.
FIG. 3 is a flowchart illustrating a process in which a diagnosis system trains a diagnostic neural network using a plurality of biometric images for training in accordance with an embodiment of the present disclosure.
FIG. 4 is a diagram illustrating an example in which two noise-added images are combined and used for learning.
FIG. 5 is a flowchart illustrating a process of diagnosing a diagnostic object biometric image by a diagnosis system in accordance with an embodiment of the present disclosure.
FIG. 6 is a diagram for explaining a process of performing diagnosis using two continuously photographed diagnostic object biometric images.
FIG. 7 is a diagram for explaining an overall structure of a diagnostic neural network for segmentation in accordance with an embodiment of the present disclosure.
FIG. 8 is a diagram for explaining an overall structure of a segmentation architecture in accordance with an embodiment of the present disclosure.
FIG. 9 is a diagram illustrating a specific example of a diagnostic neural network for segmentation in accordance with an embodiment of the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. The terms are used only for the purpose of distinguishing one component from another.

The terms used in the present application are used only to describe a particular embodiment and are not intended to limit the present disclosure. Singular expressions include plural expressions unless the context clearly means otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail centering on embodiments of the present disclosure. Like reference numerals in each figure indicate like members.

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present invention, if it is determined that a detailed description of related known technologies may obscure the gist of the present disclosure, the detailed description will be omitted.

Terms such as first and second may be used to describe various components, but the components should not be limited by the terms. The terms are used only for the purpose of distinguishing one component from another.

The terms used in the present application are used only to describe a particular embodiment and are not intended to limit the present disclosure. Singular expressions include plural expressions unless the context clearly means otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail centering on embodiments of the present disclosure. Like reference numerals in each figure indicate like members.

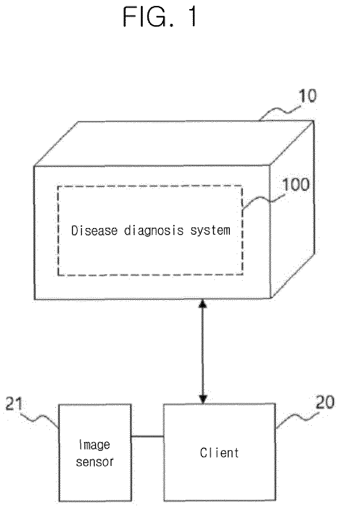

FIG. 1 is a diagram illustrating an operating environment of a method of training a neural network using a multi-phase biometric image and diagnosing a disease using the same according to the technical idea of the present disclosure. Referring to FIG. 1, the method according to the technical idea of the present disclosure may be performed by a diagnosis system 100.

The diagnosis system 100 according to the technical idea of the present disclosure may be a computing system. Alternatively, it may be installed in a predetermined server 10 to implement the technical idea of the present disclosure. The server 10 refers to a data processing device having an arithmetic capability for implementing the technical idea of the present disclosure,and in general, an average expert in the technical field of the present disclosure may easily infer that any device capable of performing a specific service, such as a personal computer, mobile terminal, etc., not just a data processing device accessible to a client (terminal 20) via a network, may be defined as a server.

The server 10 may include a processor and a storage device. The processor may refer to an arithmetic device capable of driving a program for implementing the technical idea of the present disclosure. The storage device may refer to a data storage means capable of storing programs and various data necessary for implementing the technical idea of the present disclosure, and may be implemented as a plurality of storage means according to embodiments. In addition, the storage device may include not only the main memory device included in the server 10, but also a temporary storage device or memory that may be included in the processor.

When the diagnosis system 100 is implemented by being included in a predetermined server 10, the diagnosis system 100 may communicate with at least one client (e.g., 20) accessible to the server 10. In this case, the client (e.g., 20) may transmit the biometric image to the diagnosis system 100, and the diagnosis system 100 may perform diagnosis according to the technical idea of the present disclosure on the transmitted biometric image. In addition, diagnosis results may be transmitted to the client (e.g., 20).

The diagnosis system 100 may train a neural network for diagnosis of biometric images using a plurality of biometric images for training, and after training the neural network, it is possible to predict a diagnosis result for an diagnostic object image provided by the client 20 using the trained neural network.

The client 20 may include an image sensor 21. The image sensor 21 may be a sensor for photographing or scanning a biometric image. The image sensor 21 may continuously photograph a biometric image. For example, the image sensor 21 may photograph a biometric image at 60 frames per second.

In an embodiment, the image sensor 21 may be a camera.

In another embodiment, the image sensor 21 may be a microscope. In this case, the image sensor 21 may enlarge and photograph a specific part of the biological slide, and a diagnoser who wants to diagnose a biometric image using the client 20 may perform diagnosis on a specific area of the biometric slide by moving the imaging area on the slide.

Although the disease diagnosis system 100 is shown as being implemented as any one physical device in FIG. 1, an average expert in the technical field of the present disclosure may easily infer that a plurality of physical devices may be organically combined as needed to implement the diagnosis system 100 according to the technical idea of the present disclosure.

In addition, according to embodiments, the disease diagnosis system 100 may be implemented in a form in which it is directly installed and operated in the client 20 instead of the server 10.

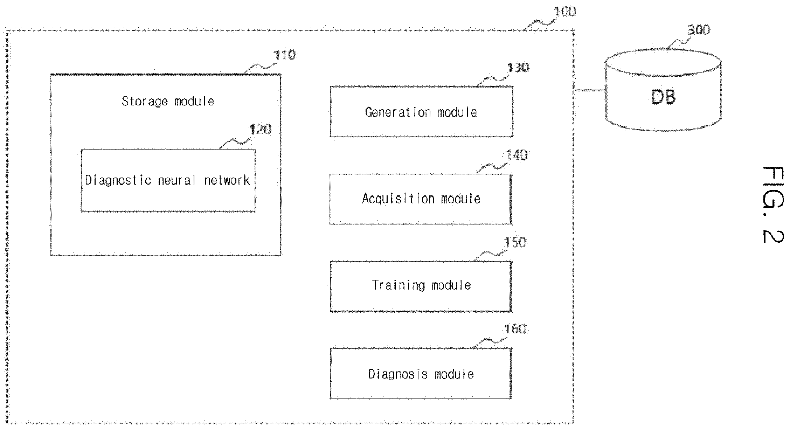

FIG. 2 is a diagram illustrating a schematic configuration of a disease diagnosis system 100 in accordance with an embodiment of the present disclosure.

Referring to FIG. 2, the system 100 may include a storage module 110, a generation module 130, an acquisition module 140, a training module 150, and a diagnosis module 160. Depending on the embodiment of the present disclosure, some of the above-described components may not necessarily correspond to components essential to the implementation of the present disclosure, and, the diagnosis system 100 may include more components than these depending on the embodiment. For example, the system 100 may further include a control module (not shown) for controlling functions and/or resources of other components (e.g., storage module 110, generation module 130, acquisition module 140, training module 150, diagnosis module 160, etc.) of the system 100. In addition, according to embodiments, the system 100 may further include a database (DB) 300 for storing various information and/or data necessary for implementing the technical idea of the present disclosure.

The system 100 may refer to a logical configuration having hardware resources and/or software required to implement the technical idea of the present disclosure, but does not necessarily refer to a single physical component or a single device. In other words, the system 100 may refer to a logical combination of hardware and/or software provided to implement the technical idea of the present disclosure, and if necessary, may be implemented as a set of logical components for implementing the technical idea of the present disclosure by being installed in devices spaced apart from each other and performing respective functions. In addition, the system 100 may refer to a set of components implemented separately for each function or role to implement the technical idea of the present disclosure. For example, each of the storage module 110, the generation module 130, the acquisition module 140, the training module 150, and the diagnosis module 160 may be located in different physical devices, and may also be located on the same physical device. In addition, depending on embodiments, a combination of software and/or hardware constituting each of the storage module 110, the generation module 130, the acquisition module 140, the training module 150 and the diagnosis module 160 may be also located in different physical devices, and the components located in different physical devices may be organically combined with each other to implement each of the modules.

In addition, in the present specification, the term module may refer to a functional and structural combination of hardware for carrying out the technical idea of the present disclosure and software for driving the hardware. For example, the module may refer to a logical unit of a predetermined code and a hardware resource for executing the predetermined code, and it may be easily inferred to an average expert in the field of the present disclosure that it does not necessarily mean physically connected code or one kind of hardware.

The DB 200 may store a plurality of biometric images for training. The biometric images for training may be various biometric images including tissue images and biopsy images.

In addition, the biometric image for training may be a biometric slide or may be a patch that is a divided part of the biometric slide.

Each of the plurality of biometric images for training may be labeled with disease diagnosis results. The labeled diagnosis result may be of the same kind as the diagnosis result diagnosed by the disease diagnosis system 100. The diagnostic results labeled in each biometric image for training may be judgment results for diseases made by medical professionals based on the biometric images in advance.

For example, when the disease diagnosis system 100 is a system for predicting classification results for whether a disease has onset, the diagnosis results labeled in each of the plurality of biometric images for training may also be for whether a disease has onset. Alternatively, depending on embodiments, the diagnosis result of the biometric image may include, for example, whether or not a specific disease has been manifested (negative/positive) as well as the degree of progression (or probability corresponding to the degree of progression) of the specific disease. For example, when the technical idea of the present disclosure is used for the diagnosis of prostate cancer, a Gleason pattern or Gleason score, which is an index indicating the degree of progression of prostate cancer, may be included in the diagnosis result. For example, the Gleason score has a value of 2 to 10, and a value of 6 to 10 may typically be considered cancer, and a larger number indicates a severe degree of prostate cancer expression. Gleason patterns can be classified into classes from 1 to 5.

If the disease diagnosis system 100 is a system that predicts a disease onset region or a diagnosis result in pixel units (i.e., a system that performs segmentation), a diagnosis result labeled on each of the plurality of biometric images for training may also relate to a disease onset region or a diagnosis result in pixel units.

Meanwhile, the disease may be prostate cancer, and hereinafter, although prostate cancer will be mainly described, those skilled in the art will easily understand that the technical idea of the present disclosure is not limited to prostate cancer.

The diagnostic neural network 120 may be stored in the storage module 110, and the diagnostic neural network 120 may be generated by the generation module 130. The diagnostic neural network 120 may be a neural network for predicting a diagnosis result for a predetermined disease using a biometric image.

The storage module 110 may refer to a data storage means capable of storing the diagnostic neural network 120, and may be implemented as a plurality of storage means depending on embodiments. In addition, the storage module 110 may include not only a main storage device included in the server 10, but also a temporary storage device or a memory.

The diagnostic neural network 120 may be a neural network for diagnosing a biometric image of a patch unit. In this case, the patch may be a partial image in which a predetermined slide is divided into a predetermined size. In other words, one slide may be divided into a plurality of patches, and the diagnostic neural network 120 may receive a patch to perform a patch level diagnosis. Before performing the diagnosis, the diagnostic neural network 120 may be pre-trained by the training module 150.

Meanwhile, the information output by the diagnostic neural network 120 may be information enabling determination of whether a specific disease (e.g., a particular type of cancer) has been manifested in a tissue corresponding to the patch or a region in which a specific disease has onset. For example, the information output by the diagnostic neural network 160 may be information indicating a probability of whether a particular disease (e.g., a particular type of cancer) has been manifested in the tissue corresponding to the patch. In this case, when the probability value output by the diagnostic neural network 120 is greater than or equal to a specific reference value (threshold value), it may be determined that a disease (e.g., prostate cancer) has been manifested in the patch. Depending on embodiments, the diagnostic neural network 120 may output information indicating the degree of progression of a particular disease (or a probability corresponding to the degree of progression) as well as whether or not a specific disease has been manifested. For example, when the technical idea of the present disclosure is used for the diagnosis of prostate cancer, a Gleason pattern or a Gleason score, which is an index indicating the degree of progression of prostate cancer, may be included in the information output by the diagnostic neural network 120. Meanwhile, the threshold value used by the diagnostic neural network 120 may be variously set, and according to the threshold value, a specific patch may be judged as a patch in which a disease has been manifested, i.e., a disease patch, or a normal patch.

Alternatively, the diagnostic neural network 120 may be a neural network for receiving a patch and predicting a classification result for the disease for each pixel constituting the patch. The diagnostic neural network 120 outputting classification results for each pixel may perform segmentation to classify disease onset regions. At this time, the diagnostic neural network 120 may output whether or not a disease has onset or the probability of disease onset for each pixel constituting the patch, and a region constituted by a pixel determined to have an onset or a pixel having an onset probability exceeding a predetermined threshold value may be determined as an onset region.

According to an embodiment, the diagnostic neural network 120 may predict and output whether the disease has onset (or probability of onset of a disease) and/or a degree of disease progression (or probability corresponding to the degree of progression) for each pixel.

Meanwhile, before performing segmentation (i.e., prediction of diagnosis results for each pixel), the diagnostic neural network 120 may be trained in advance by the training module 150.

In the present specification, a neural network may refer to a set of information representing a series of design matters defining a neural network. In an embodiment, the diagnostic neural network 120 may be a convolution neural network.

As is well known, the convolution neural network may include an input layer, a plurality of hidden layers, and an output layer. Each of the plurality of hidden layers may include a convolution layer and a pooling layer (or subsampling layer).

The convolution neural network may be defined by functions, filters, strides, weight factors, and the like for defining each of these layers. In addition, the output layer may be defined as a fully connected FeedForward layer.

The design details for each layer constituting the convolution neural network are well known. For example, known functions may be used for each of the convolution function, pooling function, and activation function for defining the plurality of layers, the number of layers to be included in the plurality of layers, and separately defined functions may be used to implement the technical idea of the present disclosure.

An example of a convolution function is a discrete convolution sum. As an example of a pooling function, max pooling, average pooling, and the like may be used. An example of the activation function may be a sigmoid, a tangent hyperbolic (tanh), a rectified linear unit (ReLU) and the like.

When the design of the convolution neural network is defined, the convolution neural network in which the design is defined may be stored in the storage device. And when the convolution neural network is pre-trained, a weight factor corresponding to each layer may be specified.

In other words, the training of the convolution neural network may refer to a process in which the weight factors of each layer are determined. In addition, when the convolution neural network is trained, the trained convolution neural network may receive input data through an input layer and output output data through a predefined output layer.

The neural network according to an embodiment of the present disclosure may be defined by selecting any one or a plurality of widely known design items as described above, and a proprietary design may be defined for the neural network.

Referring again to FIG. 2, the acquisition module 140 may acquire training data for machine learning for the diagnostic neural network 120. In other words, the acquisition module 140 may acquire a plurality of biometric images for training, and each of the plurality of biometric images for training may be labeled with corresponding diagnosis results. For example, the acquisition module 140 may acquire a biometric image for training from the DB 200.

In addition, in the present specification, an example in which the technical idea of the present disclosure is applied to prostate cancer has been mainly described, but when the technical idea of the present disclosure is applied not only to a specific tissue but also to other diseases for which it is necessary to diagnose the specific tissue in consideration of the state of the tissue surrounding the tissue, an average expert in the field of the present disclosure may easily infer that an accurate diagnosis may be possible.

Meanwhile, the training module 150 may train the diagnostic neural network 120 using the plurality of training bioimages.

More specifically, the training module 150, for each of the plurality of biometric images for training, may generate K (where K is an integer of 2 or more) noise-inserted images corresponding to the biometric images for training by inserting noises having different characteristics into the biometric images for training, and train the diagnostic neural network by inputting K noise-inserted images corresponding to the biometric images for training to the diagnostic neural network.

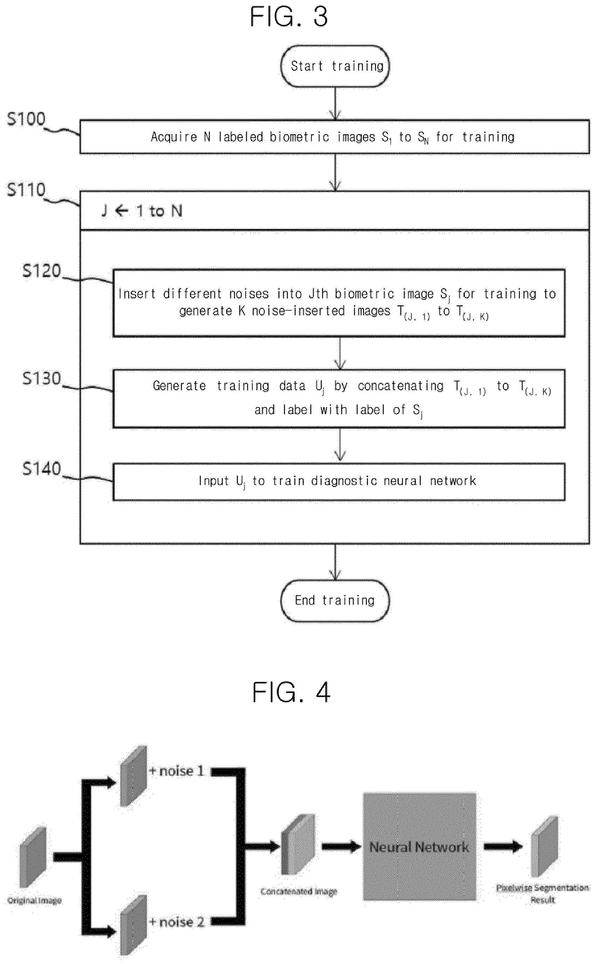

FIG. 3 is a flowchart illustrating a process in which a diagnosis system 100 trains a diagnostic neural network 120 using a plurality of biometric images for training in accordance with an embodiment of the present disclosure.

Referring to FIG. 3, the acquisition module 140 of the diagnosis system 100 may acquire biometric images for training of N (N is an integer of 2 or more) in which the diagnosis result for the disease is labeled (S100).

Thereafter, the diagnosis module 150 of the diagnosis system 100 may perform a process of S120 to S150 for each of the N biometric images for training (S110).

In the case of a training process through the Jth biometric image Sⱼ for training, in step S120, by inserting noises with different characteristics into biometric image Sⱼ for training, K (where K is an integer of 2 or more) noise-inserted images T_{(J, 1)} to T_{(J, K)} corresponding to the biometric image Sⱼ for training may be generated (S120).

For example, noise-inserted images T_{(J, 1)} to T_{(J, K)} may be an image in which Gaussian noise with different averages and/or variances is added to the biometric image for training Sⱼ. When Gaussian noise is added, various parameters may be modified in addition to the average and/or variance, and there may be embodiments in which various types of noises are added to the biometric image in addition to Gaussian noise.

In step S130, one training data Uⱼ corresponding to the biometric image Sⱼ for training may be generated by concatenating all K noise-inserted images T_{(J, 1)} to T_{(J, K )} corresponding to the biometric image Sⱼ for training. At this time, the training data Uⱼ may be labeled with a label of the biometric image Sⱼ for training (S130).

In step S140, training data Uⱼ corresponding to the biometric image Sⱼ for training may be input to the diagnostic neural network 120 to train the diagnostic neural network 120.

FIG. 4 is a diagram illustrating an example in which two noise-inserted images generated by inserting two different noises to one biometric image for training are combined and used for learning (i.e., an example in which K is 2).

As shown in Figure 4, noise 1 and noise 2 may be added to the original image to be learned to generate two different noise-inserted images, and after combining them, they may be input to the diagnostic neural network 120 to perform training.

Referring again to FIG. 2, the diagnosis module 160 may obtain K diagnostic object biometric images continuously photographed, and input K diagnostic object biometric images to the diagnostic neural network 120 trained by the training module 150 to predict the diagnosis result for the disease. In an embodiment, the K diagnostic object biometric images may be biometric images photographed by the image sensor 21.

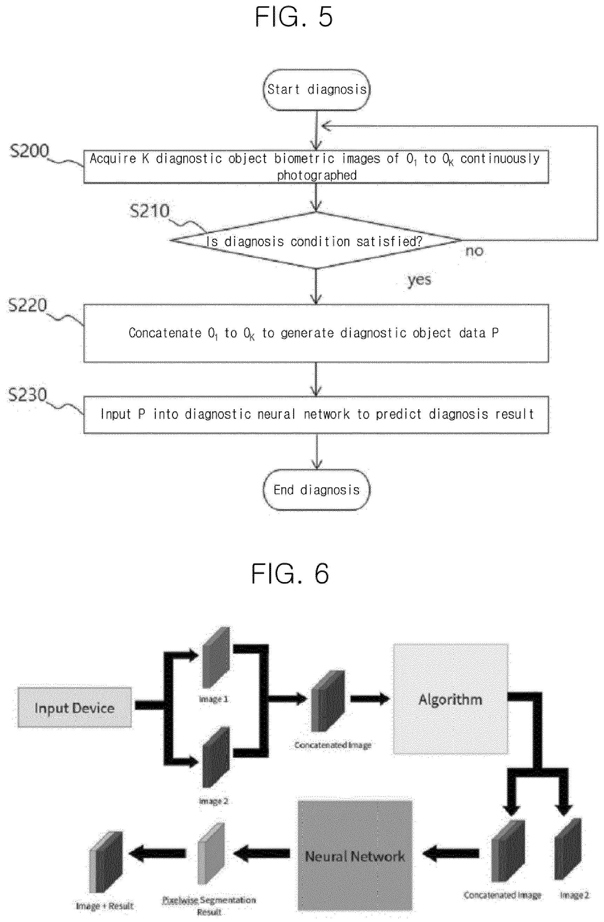

FIG. 5 is a flowchart illustrating a process of diagnosing a diagnostic object biometric image by a diagnosis system 100 in accordance with an embodiment of the present disclosure.

Referring to FIG. 5, the diagnosis module 160 may acquire continuously photographed K diagnostic object biometric images O₁ to O_{K} (S200).

The diagnosis module 160 may determine whether a predetermined diagnosis condition is satisfied based on the K diagnostic object biometric images O₁ to O_{K} (S210).

In an embodiment, the diagnosis module 160 may determine whether the image sensor 21 photographing the diagnostic object image is moving based on the K diagnostic object biometric images, and when it is determined that the image sensor 21 is not moving, it may be determined that the diagnosis condition is satisfied. In other words, the diagnosis module 160 may determine that the diagnosis condition is satisfied when the image sensor 21 is in a state of continuously photographing a specific part of the biometric image in a stationary state rather than moving.

For example, the diagnosis module 160 may calculate a numerical value or numerical values indicating a relationship between two consecutive images, and determine that it is not moving when the calculated numerical value or numerical values are within a predetermined limit range.

Alternatively, according to an embodiment, the diagnosis module 160 may determine that the diagnosis condition is satisfied when it is determined that the K diagnostic object biometric images are photographing biological tissues while the image sensor 21 is not moving. In addition, various diagnosis conditions may be set in advance.

If the diagnostic condition is not satisfied, K biometric images taken subsequently may be obtained without diagnosis of the disease being performed (see S210 and S200).

If the diagnosis condition is satisfied, the diagnosis module 160 may generate diagnostic object data P by concatenating K diagnostic object biometric imagea O₁ to O_{K} (S220), and may predict a diagnosis result for a disease by inputting diagnostic object data P to the diagnostic neural network 120.

FIG. 6 is a diagram for explaining a process of performing diagnosis using two continuously photographed diagnostic object biometric images.

As shown in FIG. 6, two diagnostic object images (i.e., image 1 and image 2) obtained from an input device (e.g., image sensor 21) may be combined with each other and input to an algorithm for determining whether diagnosis conditions are satisfied. If the diagnostic condition is satisfied, the combined data (i.e., the diagnostic object data) may be input to the trained diagnostic neural network 120, and the diagnostic neural network 120 may predict the diagnosis result.

As described above, according to an embodiment, the diagnostic neural network 12 may be a segmentation neural network that performs segmentation to specify an onset region of a disease in a biometric image (for example, a patch), and to this end, the segmentation neural network may have a very unique structure.

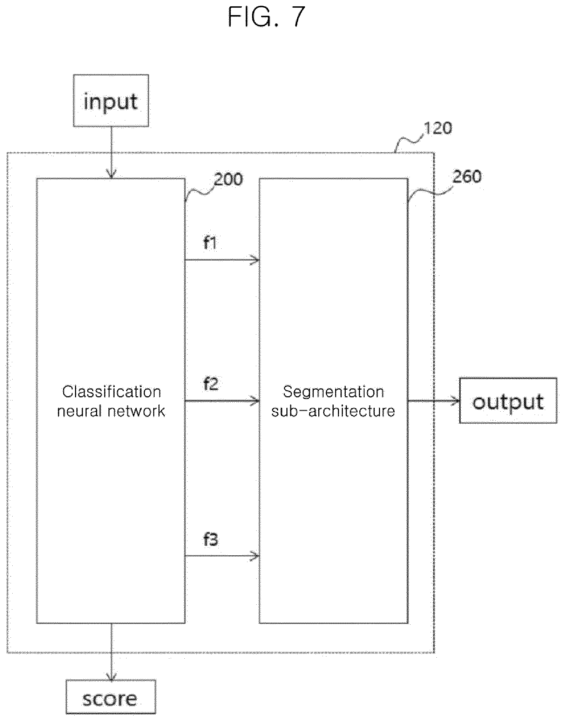

The diagnostic neural network 120 for segmentation according to an embodiment of the present disclosure may be based on a neural network ('classification neural network' to be described later) that performs classification to determine whether a disease exists in a biometric image, and may be implemented in a form in which a sub-architecture for segmentation is combined. The structure of such a diagnostic neural network 120 is shown in FIG. 7.

FIG. 7 is a diagram for explaining an overall structure of a diagnostic neural network 120 for segmentation in accordance with an embodiment of the present disclosure.

As shown in FIG. 7, the diagnostic neural network 120 according to an embodiment of the present disclosure may include a classification neural network 200 and a segmentation architecture 260.

The classification neural network 200 may receive the diagnostic object data in an input layer and output a classification result (e.g., a score as shown in FIG. 7) regarding whether or not a disease is present in a diagnostic object image corresponding to the diagnosis object data. This is called classification, and during the classification process, the classification neural network 200 may generate features for inputs (i.e., patches) as intermediate products in some hidden layers included therein. In particular, when a two-dimensional or higher matrix such as an image is received as an input, the feature generated is in the form of a two-dimensional matrix, so the term feature map is sometimes used. Meanwhile, hereinafter, a layer that generates a feature map among the hidden layers included in the classification neural network 200 will be called a feature map extraction layer.

Meanwhile, the segmentation architecture 260 may receive a feature map (for example, f1, f2, f3 shown in FIG. 4) generated from each of two or more feature map extraction layers among hidden layers included in the classification neural network 200, and may specify and output a region where a disease exists among the patches.

Although FIG. 7 shows an example in which the classification neural network 200 generates three feature maps f1, f2, and f3 in the process of performing classification, depending on the embodiment, more or fewer feature maps may be generated.

Meanwhile, according to an embodiment of the present disclosure, the classification neural network 200 that performs classification uses a known densenet, and at this time, as disclosed in the previous application, it may be designed to consider not only a specific patch to be diagnosed but also surrounding patches. In addition, various neural networks may be used, and in any case, the classification neural network 200 may be defined to receive a specific patch as an input and output a feature value corresponding to a disease onset probability of the specific patch.

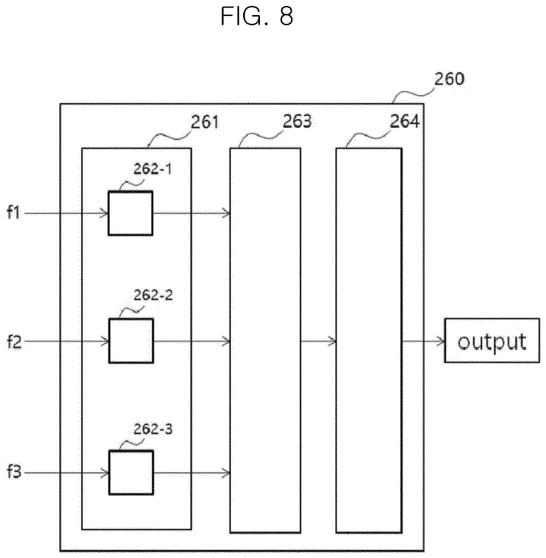

Meanwhile, FIG. 8 is a diagram for explaining the overall structure of the segmentation architecture 260.

Referring to FIG. 8, the segmentation architecture 260 may include a convolution sub-architecture 261, a segmentation sub-architecture 263, and may further include a cropping sub-architecture 264 according to an embodiment.

As described above, in the classification process performed in the classification neural network 200, feature maps (f1, f2, f3) may be generated by each feature map extraction layer, and each of the feature maps may be input to the convolution nodes 262-1 to 262-3 included in the convolution sub-architecture 261.

Each of the convolution nodes 262-1 to 262-3 corresponds to each of two or more feature map extraction layers included in the classification neural network 200, and a convolution for feature maps (f1 to f3) input from the corresponding feature map extraction layer or two or more different convolutions may be performed. Meanwhile, according to an embodiment, each of the convolution nodes 262-1 to 263-3 may perform the convolution after upscaling (e.g., upsampling) or downscaling (e.g., downsampling).

Each convolution node 262-1 to 262-3 may perform one or two or more convolutions to produce one or two or more results. According to an embodiment, the convolution performed by the convolution nodes 262-1 to 262-3 may be dialated convolution (also referred to as atrous convolution). Diallated convolution is a method of performing convolution at predetermined rate rather than extracting features from adjacent pixels, unlike conventional convolution. For example, any one of the convolution nodes (e.g., 511-2) may generate 4 convolution products (features) by performing 1x1 dialated convolution, 3x3 dialated convolution at rate 6, 3x3 dialated convolution at rate 12, 3x3 dialated convolution at rate 18.

Meanwhile, the segmentation sub-architecture 263 may specify a region where a disease exists in the patch based on a convolution result generated by the convolution sub-architecture 261.

The segmentation sub-architecture 263 may perform a predetermined operation on the convolution result generated by the convolution sub-architecture 261. The operation performed by the segmentation sub-architecture 263 may be defined as a combination of concatenation and/or convolution. According to an embodiment, concatenation and convolution may be combined in various ways.

Meanwhile, according to embodiments, the cropping sub-architecture 264 may generate a final result for segmentation by performing center-cropping on a result output from the segmentation sub-architecture 263. This is because, in the result of the convolution sub-architecture 261 and the segmentation sub-architecture 263, the central part tends to reflect the result more accurately.

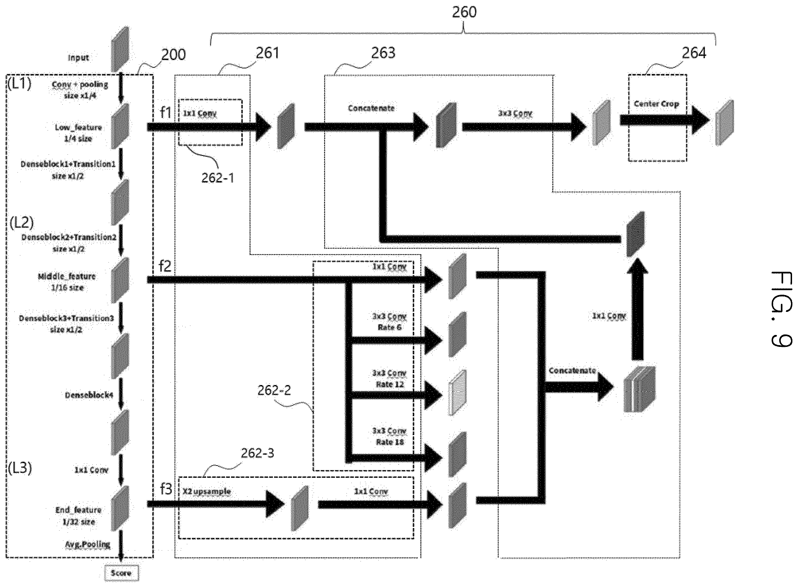

FIG. 9 is a diagram illustrating a specific example of a diagnostic neural network 120 for segmentation in accordance with an embodiment of the present disclosure.

Referring to FIG. 9, the classification neural network 200 included in the diagnostic neural network may perform classification. For classification, the classification neural network 200 may receive a patch in an input layer, and may generate a 1/4 size raw feature map f1 through convolution and pooling operations in the first feature map extraction layer L1. Subsequently, through a first dense block and a first transition operation (denseblock1, transition1) and a second dense block and a second transition operation (denseblock2, transition2), a middle feature map f2 having a size of 1/16 may be generated in the second feature map extraction layer L2. Subsequently, through the a dense block and a third transition operation (denseblock1, transition1), a fourth dense block (denseblock4), and 1x1 convolution, an end feature map f3 having a size of 1/16 may be generated in the third feature map extraction layer L3. Afterwards, a score for disease or not may be output through average pooling.

Meanwhile, the segmentation architecture 260 may perform segmentation using each feature generated by the classification neural network 200.

More specifically, each convolution node 510 included in the convolution sub-architecture 261 may perform at least one convolution of a feature map input from a corresponding feature map extraction layer in a predefined manner. In the example of FIG. 9, the first convolution node 262-1 may perform 1x1 convolution on the corresponding feature map f1 input from the first feature map extraction layer L1. The second convolution node 262-2 may perform 1x1 convolution, 3x3 dialed convolution of rate 6, 3x3 dialed convolution of rate 12, and 3x3 dialed convolution of rate 18 for the feature map f2 input from the corresponding second feature map extraction layer L2 to generate four convolution outputs (features). The third convolution node 262-3 may perform 2x upsampling on the corresponding feature map f3 input from the third feature map extraction layer L3 and then perform 1x1 convolution.

Meanwhile, the segmentation sub-architecture 263 may receive a result generated by the convolution sub-architecture 261 and perform a predefined operation. In the example of FIG. 9, the segmentation sub-architecture 263 may concatenate all convolution products (features) generated by the second convolution node 511-2 and the third convolution node 511-3 to perform a 1x1 convolution, and after combining this with the convolution result (feature) generated by the first convolution node 511-1, 3x3 convolution may be performed.

Then, center cropping may be performed in the cropping sub-architecture 264.

The accuracy of segmentation may be greatly increased by using the neural network having the technical characteristics of the present disclosure. It is empirically well known that in order to increase segmentation accuracy in a typical neural network, features should be well extracted from input data, and initial weights of the neural network should be set well before training and training should be performed. However, as described above, according to the technical idea of the present disclosure, segmentation is performed by a neural network that combines a segmentation architecture with a specific structure based on a classification neural network, and the features extracted in the patch level classification process reflect the characteristics of the input data very well. Therefore, by using this as it is in the segmentation process, there is an effect of increasing the accuracy of the segmentation.

Meanwhile, in the present specification, although an example in which the technical idea of the present disclosure is applied to prostate cancer has been mainly described, an average expert in the technical field of the present disclosure may easily infer that an accurate diagnosis may be made when the technical idea of the present disclosure is applied on not only the specific tissue, but also other diseases that need to be diagnosed in the specific tissue considering the state of the tissue surrounding the tissue.

Meanwhile, according to an embodiment, the diagnosis system 100 may include a processor and a memory storing a program executed by the processor. The processor may include a single-core CPU or a multi-core CPU. The memory may include high-speed random access memory and may also include non-volatile memory such as one or more magnetic disk storage devices, flash memory devices, or other non-volatile solid-state memory devices. Access to memory by processors and other components may be controlled by a memory controller.

Meanwhile, the diagnosis method through the multi-color model and neural network according to an embodiment of the present disclosure may be implemented in the form of computer-readable program instructions and stored in a computer-readable recording medium, and a control program and a target program according to an embodiment of the present disclosure may also be stored in a computer-readable recording medium. The computer-readable recording medium includes all types of recording devices in which data that may be read by a computer system is stored.

Program instructions recorded on a recording medium may be those specifically designed and configured for the present disclosure or may be known and available to those skilled in the software.

Examples of computer-readable recording media include magnetic media such as hard disks, floppy disks, and magnetic tapes, optical media such as CD-ROMs and DVDs, magneto-optical media such as floptic disks, and hardware devices specifically configured to store and execute program instructions such as ROM, RAM, flash memory, and the like. In addition, the computer-readable recording medium is distributed in computer systems connected through a network, so that computer-readable codes may be stored and executed in a distributed manner.

Examples of program instructions include high-level language codes that may be executed by a device that electronically processes information using an interpreter, for example, a computer, as well as machine code generated by a compiler.

The hardware device described above may be configured to act as one or more software modules to perform the operations of the present disclosure, and vice versa.

The description of the present invention described above is for illustrative purposes only, and those skilled in the art to which the present disclosure belongs will understand that it may be easily transformed into other specific forms without changing the technical idea or essential features of the present disclosure. Therefore, the embodiments described above should be understood as illustrative in all respects and not limiting. For example, each component described as a single type may be implemented in a distributed manner, and similarly, components described as distributed may also be implemented in a combined form.

The scope of the present disclosure is indicated by the claims described later rather than the detailed description above, and all changes or modifications derived from the meaning and scope of the claims and their equivalent concepts should be construed as being included in the scope of the present disclosure.

### Industrial Applicability

The present disclosure may be used for a disease diagnosis method using a neural network trained using a multi-phase biometric image and a disease diagnosis system performing the same.

## Claims

1. A method comprising:
generating, by a diagnosis system using a neural network, a diagnostic neural network for predicting a diagnosis result related to a predetermined disease using a biometric image;
acquiring, by the diagnosis system using the neural network, a plurality of biometric images for training, each of the plurality of biometric images for training being labeled with a corresponding diagnosis result for the disease, and
training, by the diagnosis system using the neural network, the diagnostic neural network using the plurality of biometric images for training,
wherein the training of the diagnostic neural network comprises, for each of the plurality of biometric images for training:
(a) generating K (where K is an integer of 2 or more) noise-inserted images corresponding to the biometric image for training by inserting noises having different characteristics into the biometric image for training; and
(b) training the diagnostic neural network by inputting the K noise-inserted images corresponding to the biometric image for training to the diagnostic neural network.

2. The method of claim 1, wherein the operation (b) comprises:
generating one training data corresponding to the biometric image for training by concatenating all the K noise-inserted images corresponding to the biometric image for training, the training data corresponding to the biometric image for training being labeled with the diagnosis result for the biometric image for training; and
training the diagnostic neural network by inputting the training data corresponding to the biometric image for training to the diagnostic neural network.

3. The method of claim 1, wherein the method further comprises a diagnosis operation, and
wherein the diagnosis operation comprises:
acquiring K diagnostic object biometric images continuously photographed by an image sensor; and
predicting a diagnosis result for the disease by inputting the K diagnostic object biometric images into the diagnostic neural network.

4. The method of claim 3, wherein the diagnosis operation further comprises determining whether the image sensor is moving based on the K diagnostic object biometric images, and
wherein the predicting of the diagnosis result for the disease by inputting the K diagnostic object biometric images into the diagnostic neural network comprises, when it is determined that the image sensor is not moving, predicting the diagnosis result for the disease by inputting the K diagnostic object biometric images into the diagnostic neural network.

5. The method of claim 2, wherein the method further comprises a diagnosis operation, and
wherein the diagnosis operation comprises:
acquiring K diagnostic object biometric images continuously photographed by an image sensor;
generating one diagnostic object data by concatenating all the K diagnostic object biometric images; and
predicting a diagnosis result for the disease by inputting the diagnostic object data into the diagnostic neural network.

6. The method of claim 5, wherein the diagnostic neural network is a segmentation neural network configured to receive the diagnostic object data in an input layer and specify a region in which the disease exists in the diagnostic object biometric image, and
wherein the segmentation neural network comprises:
a classification neural network configured to receive the diagnostic object data in an input layer and output a classification result on whether the disease exists in the biometric image; and
a segmentation architecture configured to receive a feature map generated from each of two or more feature map extraction layers among hidden layers included in the classification neural network and specify a region in which the disease exists in the biometric image.

7. The method of claim 6, wherein the segmentation architecture comprises:
a convolution sub-architecture comprising convolution nodes corresponding to the two or more feature map extraction layers, respectively, each of the convolution nodes performing convolution on a feature map input from the corresponding feature map extraction layer or two or more different convolutions; and
a segmentation sub-architecture configured to specify a region in which the disease exists in the biometric image based on a convolution result generated by the convolution sub-architecture.

8. The method of claim 1, wherein the disease is prostate cancer.

9. A computer program recorded on a non-transitory computer-readable medium for performing the method of any one of claims 1 to 8, which is installed in a data processing device.

10. A diagnosis system using a neural network, comprising:
a processor; and
a memory configured to store a computer program,
wherein the computer program, when executed by the processor, causes the diagnosis system using the neural network to perform the method of any one of claims 1 to 8.

11. A diagnosis system using a neural network, the diagnosis system comprising:
a storage module configured to store a diagnostic neural network for predicting a diagnosis result related to a predetermined disease using a biometric image;
an acquisition module configured to acquire a plurality of biometric images for training, each of the plurality of biometric images for training being labeled with a corresponding diagnosis result for the disease; and
a training module configured to train the diagnostic neural network using the plurality of biometric images for training,
wherein the training module performs, for each of the plurality of biometric images for training,
(a) generating K (where K is an integer of 2 or more) noise-inserted images corresponding to the biometric image for training by inserting noises having different characteristics into the biometric image for training; and
(b) training the diagnostic neural network by inputting the K noise-inserted images corresponding to the biometric image for training to the diagnostic neural network.

12. The diagnosis system of claim 11, wherein the operation (b) comprises:
generating one training data corresponding to the biometric image for training by concatenating all the K noise-inserted images corresponding to the biometric image for training, the training data corresponding to the biometric image for training being labeled with the diagnosis result for the biometric image for training; and
training the diagnostic neural network by inputting the training data corresponding to the biometric image for training to the diagnostic neural network.

13. The diagnosis system of claim 11, further comprising a diagnosis module configured to acquire K diagnostic object biometric images continuously photographed by an image sensor and predict a diagnosis result for the disease by inputting the K diagnostic object biometric images into the diagnostic neural network.

14. The diagnosis system of claim 13, wherein the diagnosis module is configured to determine whether the image sensor is moving based on the K diagnostic object biometric images, and
when it is determined that the image sensor is not moving, predict the diagnosis result for the disease by inputting the K diagnostic object biometric images into the diagnostic neural network.

15. The diagnosis system of claim 12, further comprising:
a diagnosis module configured to:
acquire K diagnostic object biometric images continuously photographed by an image sensor;
generate one diagnostic object data by concatenating all the K diagnostic object biometric images; and
predict a diagnosis result for the disease by inputting the diagnostic object data into the diagnostic neural network.
